Europäisches Patentamt

(19) European Patent Office

Office européen des brevets

(11) Publication number: **0 398 707**
**A1**

## (12) EUROPEAN PATENT APPLICATION

(21) Application number: **90305325.4**

(22) Date of filing: **17.05.90**

(51) Int. Cl.⁵: **C07K 7/06, A61K 39/21, G01N 33/569**

(30) Priority: **17.05.89 US 353021**

(43) Date of publication of application:
**22.11.90 Bulletin 90/47**

(84) Designated Contracting States:
**AT BE CH DE DK ES FR GB GR IT LI LU NL SE**

(71) Applicant: **SANGSTAT MEDICAL CORPORATION**
**3603 Haven Avenue, Suite D**
**Menlo Park, CA 94025(US)**

(72) Inventor: **Pouletty, Philippe J.**
**146 Park Lane**
**Atherton, California 94027(US)**
Inventor: **Sra, Kuldip**
**6137 Arlington Boulevard**
**Richmond, California 94805(US)**

(74) Representative: **Paget, Hugh Charles Edward et al**
**MEWBURN ELLIS 2 Cursitor Street**
**London EC4A 1BQ(GB)**

(54) **Treatment and diagnosis of human lymphotropic viruses.**

(57) Novel compositions are provided based on the use of a core sequence EPTAP. Oligopeptides, antibodies to the oligopeptides and anti-idiotypes are provided in diagnosis and therapy.

EP 0 398 707 A1

## TREATMENT AND DIAGNOSIS OF HUMAN LYMPHOTROPIC VIRUSES

The subject invention concerns disease diagnosis and modulation of interaction with CD5 for the treatment of lymphotropic diseases in humans.

Human lymphotropic viruses have only been found in the last decade. These viruses have proven to be associated with the induction of malignant disease, and immunodeficiency, have a complex genome, which includes genes associated with the transformation of cells. Using phase shifts and variable splicing, a number of genes in addition to the normal envelope, core and reverse transcriptase genes are encoded. Among these viruses and one that has proven very complex in its mechanism of pathogenicity is HIV.

The exact mechanism by which HIV exerts its immunosuppressive activity has not been precisely determined. There is convincing evidence that the virus has a direct cytopathic effect upon T lymphocytes. Nevertheless, the percentage of T lymphocytes actually expressing virus in the peripheral blood of infected individuals is very low (about 1 cell in $10^5$). Thus, the direct cytopathic effect of the virus cannot explain the dramatic decrease of T lymphocytes during HIV infection. Furthermore, the immuno-suppression is related not only to a quantitative cell defect, but also to qualitative abnormalities of T lymphocytes reflected by a deficient response of CD4 lymphocytes to soluble antigens. Also, T-cells are not the only lymphocyte population affected following HIV infection. There is a polyclonal B-cell activation with secretion of auto antibody in AIDS.

A major effort has been devoted to the study of the HIV envelope. The identification of CD4 as the primary cell receptor for HIV, of the in vitro immunosuppressive activity of some gp41 peptides and homology between neuroleukin and HIV envelope protein has offered some insight in the pathogenesis of AIDS. In contrast, gag encoded proteins have been given less attention. It is found that these gag sequences are highly conserved among HIV-1 isolates and between HIV-1 and HIV-2. The high level of p24/p25 antigen detected in several patients at an advanced clinical stage emphasizes the need to better understand the potential role of the core protein in the etiology of the disease.

At the present time, there is an essential need to further elucidate the process by which HIV is able to suppress the immune system in order to be able to provide various therapies. As part of this investigation, it is important to develop new techniques for diagnosis which may allow for better staging of the disease, give some direction as to therapies, as well as provide for new therapeutic opportunities.

Relevant Literature

Huang, et al., Proc. Natl. Acad. Sci. USA 84:204-208, 1987, describe the molecular cloning of Ly-1, a membrane glycoprotein of mouse T lymphocytes. Jones, et al., Nature 323:346-349, 1986, describe the CDNA sequence of the human lymphocyte glycoprotein Ti/Leu-1. Palker, et al., Journal of Immunology, 136:2393-2397, 1986, describes specific epitopes of HTLV-1 p19 core protein.

Oligopeptides having for the most part the core sequence EPTAP, antibodies to such peptides, and antibodies to the idiotopes of such antibodies are provided for modulating human lymphotropic virus interaction with T lymphocytes, diagnosing the presence of the disease and providing for therapies in modulating the indications and etiology of the disease.

Methods and compositions are provided for diagnosing and treating human viral lymphotropic diseases. The methods and compositions are predicated on the use of an oligopeptide having the sequence E/T/L-P-T-A-P, preferably having one additional P at the C-terminus (the left amino acid is the N-terminus while the right amino acid is the C-terminus) and one additional P at the N-terminus (PEPTAP). The oligopeptides by themselves or bound to other amino acids find particular application in the diagnosis and/or the prognosis of human lymphotropic diseases, as well as modulating the progression of the disease in a human host.

The region associated with EPTAP (in HIV, the lymphotropic viruses, and CD5) is rich in T and P amino acids. Of particular interest are sequences of doublets or triplets of T's and P's, the TP sequences, such as TTTPP, TTPPPTTTPP or TTPPTTT*PPPTTT, where T* may be substituted by R. These sequences will have the formula $(T_nP_p)_qT_r$, where n = 2 - 3, p = 2 - 3, g = 1 -3 and r is 0 or 1. Generally the sequence will have from 5 to 16 amino acids, usually about 10 to 14 amino acids. These sequences may be used in combination with the EPTAP sequence in diagnosis and therapy.

The primary oligopeptide sequences of the subject invention will comprise EPTAP, where the N-terminus may be extended, usually by not more than one amino acid, which will be either P or L and the C-terminus will be extended by from one to four, usually one to two amino acids, e.g. P-R/E/F-L-Q, usually by only one amino acid, where the first amino acid is P and the second amino acid is E, F, V, or R. The sequences will normally be not more than 10, usually not more than 9 amino acids. Other than the core sequence, and proline, conservative substitutions may be employed in the above indicated sequence where leucine may be replaced by isoleucine or valine and phenylalanine by tryptophan and tyrosine. The other oligopeptides, the TP sequences, may be treated in the same manner as the EPTAP sequences.

The oligopeptides may be joined to other amino acids, but these amino acids will be other than the naturally-occurring amino acids associated with EPTAP sequence or TP sequence in CD5, HIV, LAV, and HTLV-1.

The core sequence which may be bound to additional amino acids, other compounds, or solid phases for convenience; it may be used for the detection of antibodies or core antigen (by competitive immunoassay), which indicate the presence of a human lymphotropic viral disease in a human host. Various assays may be employed, which are conventionally known, where the oligopeptides described above may be joined directly or by a linking group to a label or to a solid phase (directly or indirectly by a carrier compound). Labels include enzymes, fluorescent molecules, radioisotopes, chemiluminescent molecules, particles, e.g., colloidal particles, and the like. Numerous commercial assays are available which may be employed, such as ELISA, EMIT, RIA, CEDIA, SLFIA or the like. The particular assay which is employed is not critical to this invention, and any protocol may be employed which provides the desired sensitivity. Enzymes which may find use include horseradish peroxidase, alkaline phosphatase, lysozyme, $\beta$-galactosidase, or the like. Fluorescent compounds which may find use include phycoerythrins, fluorescein, rhodamine, lathanide chelates, or the like. For exemplification of various diagnostic assays, see for example, U.S. Patent Numbers 3,791,932; 3,817,837; 4,233,402 and 4,134,792.

The subject peptides may also be used for the production of antibodies, which may find use in diagnostic assays as a reagent, where the antibodies will detect the presence of the subject sequences on other than human T lymphocytes. Alternatively, the antibodies may be used in a competition assay, competing with antibody present in serum for the subject peptides. In the latter situation, the antibodies may be labeled as described above, or a secondary antibody specific for the anti-oligopeptide may be employed. As illustrative of the situation, the subject peptides may be bound to a support and combined with serum, where any antibodies in the serum which bind to the specific peptides will bind to form immune complexes. By then adding antibody to the constant portion of the human antibodies, either light and/or heavy chain isotype specific or not, where the anti-antibodies are labeled, one can determine the level of binding of the antibodies to the surface. For example, the anti-antibodies may be mouse, rat, sheep, goat, bovine or the like.

Quite evidently, protocols may be devised for the detection of either viruses or antibodies to the virus in human blood, as blood, serum, or plasma, or in tears, CSF or other body fluid, or in tissue by immunohistochemistry. The particular manner in which the virus peptide is detected, employing the subject oligopeptides or antibodies thereto, may be varied in accordance with available equipment, ease of synthesis of reagents, and the like.

Besides antibodies which bind to the subject oligopeptides, anti-idiotype antibodies may be prepared which will mimic the conformation and binding properties of the oligopeptides of the subject invention. Modulation of the disease may be achieved by use of the subject peptide, antibodies to the subject peptide, or anti-idiotype antibodies. These various compounds may be administered in conventional ways, such as parenterally, e.g. peritoneally, intravascularly, or the like. The oligopeptide or antibodies may be administered in liposomes. The level of administration will vary widely, generally between the range of about 1 mg/Kg/day to 100 mg/Kg/day. Conveniently, the subject compositions may be administered in an appropriate vehicle, such as deionized water, saline, phosphate buffered saline, aqueous ethanol, or the like.

The subject oligopeptides share homology with sequences present in human CD5, a pan-T marker expressed at a higher level by helper T-cells, also found on some B-cells. To distinguish from binding to CD5, it may be desirable to prepare antibodies to the oligopeptides which are extended by one to three, usually one to two amino acids, which distinguish the viral sequence from the CD5 sequence. For example, with EPTAP by adding P-E, P-F, or Q-V and then using the extended oligopeptide to prepare antibodies, particularly monoclonal antibodies, antibodies may be obtained which distinguish the viral sequence from the human CD5 sequence. One would first screen the hybridoma clones for antibodies which bind strongly to EPTAP, followed by screening for antibodies which bind to EPTAPPR and discarding those antibodies. The remaining antibodies are screened for binding to one or more of the viral sequences.

Also, the subject peptides may be joined to any convenient immunogen or T-cell immunodominant sequence for preparation of antibodies. Various T-cell immunodominant sequences have been described in the literature, see, for example EP-A 87.905022.7, filed February 26, 1988. In this manner, antibodies may be produced which are specifically directed to the epitope of interest. The oligopeptide may be joined to various carriers, such as serum albumin, keyhole limpet hemocyanin, $\gamma$-globulin or the like. Methods for

3

linking the oligopeptide to the immunogen are well known and described in the literature. The resulting immunogen may then be used for immunizing a mammalian host in accordance with known ways, particularly injection, normally with adjuvant, such as Freund's adjuvant. Booster injections may be made over periods of from one to three weeks and the titer of the desired antibody assayed. The blood may then be isolated and the γ-globulin precipitated with ammonium sulfate, providing for polyclonal antibodies. For monoclonal antibodies, usually the host will be a mouse, where splenocytes will then be fused with a fusion partner, followed by cloning under limiting dilution, growing of the cells in an appropriate nutrient medium, and screening of the resulting clones. The subject oligopeptides may be used to identify the clones which have the desired specificity and affinity. The monoclonal antibodies may then be used to identify the presence of T-lymphotropic viruses in a host, where either the protein of the virus or antibodies to the virus may be detected. Where the antibodies are to be used for therapy and the antibodies are xenogenic, F-(ab')$_2$ or Fab may be used. Alternatively, the mRNA for the light and heavy chains may be isolated from the hybridoma, the sequences encoding the variable regions isolated and bound to human light and heavy constant regions. B-cells from patients with antibodies against the sequence(s) can be transformed by EBV and/or fused with human or mouse or cat myelomas to make human monoclonals.

Some antibodies may be employed that differ by a factor of at least 10, usually $10^2$-fold greater in affinity for the peptide associated with the viral protein as compared to CD5 or to the CD5 as compared to such antibodies, individually or in combination.

In order to enhance the stability of the subject compositions, they may be modified by amidification of the terminal carboxyl group, acylation, or alkylation of the terminal amino group, particularly with substituents of from about 1 to 7, more usually of from about 1 to 3 carbon atoms, additional amino acids of the non-natural D-stereoisomer, etc.

The subject compositions may be easily prepared by synthesis in accordance with conventional techniques. Automatic synthesizers are available so that the oligopeptide may be easily prepared on a commercial synthesizer. Activated derivatives of the individual amino acids are prepared and added sequentially in accordance with known techniques, using the Merrifield procedure. The individual side functional groups are protected, usually using aralkl groups for oxygen and sulfur side groups, and acyl groups for amino side groups. For a description of the synthesis of oligopeptides, see WO84/03506 and WO84/03564, which disclosure is incorporated herein by reference. The peptides may be used crude or first purified using known procedures.

The subject polypeptides may be used to produce monoclonal antibodies which are highly specific for a particular sequence or group of relaLed sequences. For example, monoclonal antibodies may be prepared which cross-react with different sequences or are specific for a particular sequence, where the sequences may differ only by one or two amino acids. Thus, the monoclonal antibodies may be able to detect the presence of any one of a group of lymphotropic or other viruses sharing the E-P-T-A-P motif. The monoclonal antibodies may be prepared to exclude binding to CD5, while binding to one or more retroviral gag sequences sharing the E-P-T-A-P motif.

The homology between human CD5 and retroviral gag sequence (p15) for EPTAP is shown in the following table.

| PROTEIN | N-TERMINAL AMINO ACID | SEQUENCE |
|---|---|---|
| CD5 | 151 | PEPTAPPRLQ |
| HTLV-III(BH10) | 453 | PEPTAPPFLQ |
| HTLVIII(BH10) | 465 | PEPTAPPEES |
| LAV-1a | 453 | PEPTAPPEESF |
| ARV-2 | 455 | PEPTAPPEESF |
| HIV-2(ROD) | 454 | LTPTAPPVDPA |
| SIV | | LTPTAPPEEPA |
| HTLV-1 | 123 | EPTAPQVL |

In addition, HTLV-III has a repeat of the sequence in P15, the following being the C-terminal sequence: QSRPEPTAPPFLQSRPEPTAPPEESFRSGVETTTPP

The homology between human CD5 and the retroviral gag sequence (p15) for the TP sequences is as follows:

| CD5 | 115 | TTPPTTRPPPTTTPEP |
|-----|-----|------------------|
| HIV | 481 | TTTPP            |

Individual or mixtures of peptides may be used to identify the presence of antibodies in sera which specifically bind to an oligopeptide comprising the E-P-T-A-P motif. Thus, one can determine the presence of antibodies which cross-react with the gag protein and CD5 by using a combination of sequences or identifying a sequence which may provide for binding to cross-reactive antibodies, e.g. P-E-P-T-A-P-P-I/V/L/M/Q-L-Q (where / interests either amino acid). Instead of peptides, antiidiotype antibodies may be employed which mimic one or more peptides.

In accordance with the subject invention, compositions are provided which may be used in the diagnosis or therapy of human lymphotropic viral diseases. The compositions may be used in diagnostic assays, for the detection of the virus, or for preparation of antibodies to the virus. In addition, the compositions may find use in the treatment of the disease, in modulating the interaction with CD5 or other components present in blood, and the core proteins of the retrovirus.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto

## Claims

1. An amino acid polypeptide comprising not more than a total of ten amino acids of the naturally occurring sequence of CD5, or p15 of HIV-1 or-2, or HTLV-1, and comprising the sequence E-P-T-A-P.

2. A polypeptide according to Claim 1, wherein said sequence comprises up to four of the naturally occurring amino acids at the C-terminus of p15.

3. A polypeptide according to Claim 2, wherein said sequence comprises E-P-T-A-P-P-F.

4. A polypeptide according to Claim 2 of the sequence P-E-P-T-A-P-P-F-L-Q, E-P-T-A-P-P-E-E-S, P-E-P-T-A-P-P-E-E-S, or P-E-P-T-A-P-P-R-L-Q.

5. A polypeptide composition comprising a polypeptide according to any one of Claims 1 to 4 and a second polypeptide of from five to 16 amino acids comprising alternating doublets or triplets of T and P.

6. A polypeptide composition wherein said second polypeptide has the formula T-T-T-P-P.

7. A monoclonal antibody which binds specifically to an amino acid polypeptide comprising not more than a total of ten amino acids of the naturally occurring sequence of p15 of HIV-1 or-2, or HTLV-1, and comprising the sequence E-P-T-A-P.

8. An antibody composition according to Claim 7, wherein said antibody specifically binds to a peptide of the sequence P-E-P-T-A-P-P-F-L-Q, P-E-P-T-A-P-P-E-E-S, P-E-P-T-A-P-P-R-L-Q, or E-P-T-A-P-P-F-L-Q.

9. An antibody composition according to Claim 7 or Claim 8, comprising in addition a monoclonal antibody to a second polypeptide of from five to 16 amino acids comprising alternating doublets or triplets of T and P.

10. An immunogen comprising an amino acid polypeptide comprising not more than a total of ten amino acids of the naturally occurring sequence of CD5, or p15 of HIV-1 or-2, or HTLV-1, and comprising the sequence E-P-T-A-P covalently bonded to compound or liposome other than CD5 or a capsid protein of a lymphotropic retrovirus.

11. In a method for detecting the presence of a human lymphotropic retrovirus in a body fluid, the improvement which comprises employing at least one of a polypeptide comprising not more than a total of ten amino acids of the naturally occurring sequence of p15 capsid protein of HIV-1 or-2, or HTLV-1, and comprising the sequence E-P-T-A-P or a monoclonal antibody which binds specifically to an amino acid polypeptide comprising not more than a total of nine amino acids of the naturally occurring sequence of p15 of HIV-1 or-2, or HTLV-1, and comprising the sequence E-P-T-A-P, and said antibody has an affinity for said polypeptide at least 10 greater than the affinity for the sequence comprising E-P-T-A-P-P-R-L-Q.

12. A process for preparing an amino acid polypeptide or polypeptide composition according to any one of Claims 1 to 6, or an antibody or antibody composition according to any one of Claims 7 to 9 or an immunogen according to Claim 10.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-107053 (JAPANESE FOUNDATION FOR CANCER RESEARCH)<br>* abstract; claim 8 * | 1, 7,<br>10, 12 | C07K7/06<br>A61K39/21<br>G01N33/569 |
| A | EP-A-230222 (F. HOFFMANN-LA ROCHE &CO.)<br>* claims 7, 34, 38, 40; figure 12 * | 1, 3-4,<br>7 | |
| D,A | Proceedings of the National Academy of Sciences, USA<br>vol. 84, January 1987, Washington D.C.<br>pages 204 - 208; Huang H.S. et al:<br>"Molecular cloning of Ly-1, a membrane glycoprotein of mouse T lymphocytes and a subset of B cells: Molecular homology to its human counterpart Leu-1/T1 (CD5)"<br>* figure 4 * | 1-6 | |
| | | | TECHNICAL FIELDS SEARCHED (Int. Cl.5)<br><br>C07K<br>A61K |

The present search report has been drawn up for all claims

| Place of search | Date of completion of the search | Examiner |
|---|---|---|
| THE HAGUE | 26 JULY 1990 | BEVAN S.R. |

EPO FORM 1503 03.82 (P0401)